# FASCICULE DE BREVET EUROPEEN

(11) **EP 0 647 443 B1**
(45) Date de publication et mention de la délivrance du brevet: **28.08.1996**
(21) Numéro de dépôt: 94402156.7
(22) Date de dépôt: 28.09.1994
(51) Int. Cl.: A61K 7/00

(54) **Emulsion huile-dans-eau utilisable pour l'obtention d'une crème**
Öl in wasser Emulsionen verwendbar für die Herstellung von einer Creme
Oil in water emulsion usable for the preparation of a cream

(30) Priorité: 08.10.1993 FR 9311995
(43) Date de publication de la demande: 12.04.1995
(73) Titulaire: L'OREAL, F-75008 Paris (FR)
(72) Inventeur: Candau, Didier, F-91570 Bievres (FR); Simon, Pascal, F-94400 Vitry sur Seine (FR)
(74) Mandataire: Lhoste, Catherine

(56) Documents cités:
- EP-A- 0 428 157
- EP-A- 0 458 600
- WO-A-92/01508
- FR-A- 2 698 004
- DATABASE WPI Week 8702, Derwent Publications Ltd., London, GB; AN 87-012136 & JP-A-61 271 205 (KANEBO KK) 1 Décembre 1986
- DATABASE WPI Week 9243, Derwent Publications Ltd., London, GB; AN 92-352737 & JP-A-4 253 903 (KANEBO LTD) 9 Septembre 1992
- PATENT ABSTRACTS OF JAPAN vol. 15, no. 472 (C-0889) & JP-A-03 000 710 (LION CORPORATION) 02 Septembre 1991
- PATENT ABSTRACTS OF JAPAN vol. 18, no. 1 (C-1148) & JP-A-52 046 821 (SHISEIDO COMPANY LIMITED) 24 Septembre 1993

## Description

La présente invention se rapporte à une nouvelle émulsion huile-dans-eau d'aspect crémeux, blanc et brillant, destinée notamment à la préparation de crèmes pour le soin de la peau (corps, visage) et/ou le maquillage du visage mais aussi pour le traitement de certaines maladies de peau. Si besoin est, ces crèmes peuvent être colorées à l'aide de pigment en vue de réaliser des fonds de teint. Ces crèmes sont donc destinées à la cosmétologie et à la dermatologie.

L'invention se rapporte aussi à l'utilisation d'un premier et d'un second esters comme système émulsionnant.

Il est connu de l'homme du métier d'utiliser un système émulsionnant pour stabiliser ensemble une phase aqueuse et une phase huileuse, et ce afin d'obtenir notamment des crèmes. Le choix des émulsionnants revêt une importance particulière pour la fabrication de crèmes bien tolérées par le consommateur.

Parmi les systèmes émulsionnants, on connait l'utilisation de dérivés issus du pétrole. Mais, bien que n'étant pas nocifs et étant correctement tolérés par la peau, ces dérivés constituent une matière première, pour préparer les émulsions, non biodégradable et sont dépendants de la quantité en réserve naturelle de pétrole. Par ailleurs, l'emploi de tels dérivés dans la fabrication d'émulsions entraîne l'utilisation de solvants organiques présentant une toxicité plus ou moins élevée pour la peau.

Le brevet japonais JP-A-61271205 divulgue une composition cosmétique contenant un ester d'acide gras et de glucose ou de sucrose comme agent émulsionnant. Un tel agent émulsionnant présente l'avantage d'être mieux toléré par les peaux sensibles que les dérivés du pétrole ; il est non irritant et respectueux du pH et du film hydro-lipidique de la peau. En revanche, il possède l'inconvénient de donner des crèmes rugueuses lors de l'application sur la peau les rendant désagréables pour l'utilisateur.

Il est également connu (brevet japonais JP-A-03193210) d'utiliser un ester d'acide gras et de saccharose comme agent émulsionnant pour préparer des émulsions. Mais l'utilisation de tels agents présente l'inconvénient de donner des émulsions thermiquement fragiles. En effet, ces agents étant plus facilement susceptibles de se dégrader à des températures allant de 50° à 70° C usuellement utilisées lors de la fabrication des émulsions, donnent des produits finis couleur caramel. En outre, cette couleur se développe naturellement lorsque ces esters de saccharose sont mis en solution aqueuse ou en émulsion. Cette couleur devient rédhibitoire dans la mesure où l'on désire avant tout des produits blancs, mieux appréciés du consommateur.

L'émulsion selon l'invention permet, en particulier, de pallier les problèmes mentionnés précédemment. Notamment, il a été découvert de façon surprenante que l'association d'au moins un premier ester choisi parmi les esters d'acides gras et de glucose et les esters d'acide gras et d'alkylglucose et au moins un second ester d'acide gras et de saccharose, comme système émulsionnant dans une émulsion huile-dans-eau, permet d'obtenir des crèmes blanches, brillantes et moins rugueuses à l'application et ce quelle que soit leur composition. En outre, cette émulsion a l'avantage de comprendre des composés biodégradables et renouvelables. Par ailleurs, la température de fabrication de cette émulsion n'affecte plus la couleur qui reste blanche.

L'invention se rapporte à une émulsion huile-dans-eau comprenant une phase huileuse dispersée dans une phase aqueuse, un système émulsionnant, le système émulsionnant contenant au moins un premier ester choisi parmi les esters d'acide gras et de glucose et les esters d'acide gras et d'alkylglucose et au moins un second ester d'acide gras et de saccharose.

Les proportions de la phase aqueuse peuvent aller de 40 % à 94 % en poids par rapport au poids total de l'émulsion. Les proportions de la phase huileuse peuvent aller de 5 % à 50 %, et de préférence de 10 % à 30 %, en poids par rapport au poids total de l'émulsion. Le système émulsionnant peut représenter de 1 % à 10 % en poids par rapport au poids total de l'émulsion.

Selon une variante préférentielle, l'émulsion peut comprendre de 66 % à 86 % en poids de phase aqueuse, de 10 % à 30 % en poids de phase huileuse, de 4 % à 6 % en poids de système émulsionnant, de 0 % à 10 % en poids d'additifs par rapport au poids total de l'émulsion.

Le rapport en poids premier ester/second ester est choisi de préférence dans les rapports allant de 70/30 à 30/70. Ce rapport est de préférence de 50/50. Cependant, on peut envisager des rapports allant de 1/99 à 99/1.

Le premier ester d'acide gras peut être choisi parmi les composés obtenus par réaction d'un acide gras à chaîne saturée ou insaturée ayant de 12 à 22 atomes de carbone et de préférence 16 à 20 atomes de carbone avec un glucose ou avec un alkylglucose dont le groupement alkyle contient de 1 à 6 atomes de carbone.

Les esters d'acide gras et d'alkylglucose sont des éthers en C₁ du glucose dont la chaîne alkyle comprend de 1 à 6 atomes de carbone.

Le premier ester d'acide gras peut contenir un mélange de dérivés mono-, di-, tri-, tétra-ester avec une proportion pouvant être d'au moins 50 % en poids de dérivés mono- et diester et n'excédant pas 95 % en poids de dérivés mono-ester par rapport au poids total du mélange.

Le second ester peut être choisi parmi les composés obtenus par réaction d'un acide gras à chaîne carbonée saturée ou insaturée ayant de 12 à 22 atomes de carbone, et de préférence de 16 à 20 atomes de carbone, avec du saccharose.

Pour des esters en C₁₁, C₁₀, on obtient plutôt une solution détergente et pour des esters en C₂₃, C₂₄, on obtient un produit plutôt solide.

Le second ester d'acide gras peut contenir un mélange de dérivés mono-, di-, tri-, tétra- et polyester avec une proportion en dérivés mono-ester pouvant aller de 30 % à 95 % en poids par rapport au poids total du mélange.

Le premier ester peut être choisi parmi le palmitate de glucose, les sesquistéarates d'alkylglucose comme le sesquistéarate de méthylglucose, les palmitates d'alkylglucose comme le palmitate de méthylglucose ou d'éthylglucose.

L'invention se rapporte aussi à une utilisation d'au moins un premier ester choisi parmi les esters d'acide gras et de glucose et les esters d'acide gras et d'alkylglucose et au moins un second ester d'acide gras et de saccharose dans une émulsion huile-dans-eau, comme système émulsionnant.

Le second ester peut être choisi parmi le palmito-stéarate de saccharose, le stéarate de saccharose et le mono laurate de saccharose.

Comme phase huileuse, on peut utiliser une ou plusieurs huiles choisies parmi des organopolysiloxanes, des huiles minérales, organiques, synthétiques ou végétales, notamment l'huile de vaseline, de jojoba, de tournesol, le perhydrosqualène de synthèse, l'isostéarate d'isostéaryle, le myristate d'isopropyle.

L'émulsion de l'invention peut également contenir des agents gélifiants choisis parmi les acides polyacryliques neutralisés par une base, les gommes naturelles formées de polysaccharides (guar, xanthane). La proportion en agents gélifiants représente de 0,01 % à 1 % en poids par rapport au poids total de l'émulsion.

L'émulsion selon l'invention peut encore contenir un additif choisi parmi les hydratants (polyols), les vitamines, les filtres UV, les conservateurs, les parfums, les anti-oxydants. Parmi les polyols, on peut utiliser le glycérol.

Les proportions en hydratants, vitamines, filtres UV, vont respectivement de 0,1 % à 10 % en poids, de 0,1 % à 5 % en poids et de 0,5 % à 5 % en poids par rapport au poids total de l'émulsion.

D'autres additifs peuvent être envisagés comme les pigments organiques ou inorganiques et les co-émulsionnants. Comme co-émulsionnants, on peut utiliser des alcools gras ayant de 12 à 24 atomes de carbone, et de préférence de 16 à 22 atomes de carbone. La proportion en alcool gras va de 1 % à 5 % en poids par rapport au poids total de l'émulsion. Comme alcool gras, on peut citer l'alcool cétylique, l'alcool béhénique...

Dans le cas des esters d'acide gras et de glucose, l'acide gras peut être fixé sur le résidu glucose indifféremment en position C₂, C₃, C₄ ou C₆. Ceci permet de donner un mélange de mono-, di-, tri- et tétraester. Ce mélange peut comprendre au moins 50 % en poids de dérivés mono- et diester mais n'excède pas 95 % en poids de dérivés monoester par rapport au poids total du mélange. Ces proportions en ces dérivés d'ester permettent d'obtenir une émulsion huile-dans-eau.

Les esters d'acide gras et de glucose utilisés selon l'invention sont par exemple choisis parmi les "GLUCATE SS" vendus par la Société AMERCHOL, les "GRILLOCOSE PS" vendus par la Société GRILLO-WERKE, ou le "BIOSURF 16" vendu par la Société NOVO.

La chaîne grasse des esters d'acide gras de saccharose est une chaîne saturée ou insaturée qui comprend de 12 à 22 atomes de carbone.

L'acide gras peut être fixé sur le résidu saccharose en position C₂, C₃, C₄ ou C₆ du résidu glucose ou en position C₂, C₃, C₅ ou C₆ du résidu fructose. Ceci permet d'obtenir un mélange de mono-, di-, tri-, tétra et polyester. Les ester d'acide gras de saccharose utilisés sont par exemple choisis parmi les crodesta F160, F140, F110, F90, F70, SL40 vendus par la Société CRODA ou parmi les Ryoto sugar esters vendus par la Société MITSUBISHI.

La proportion préférentielle du mélange premier ester-second ester est d'environ 5 % en poids par rapport au poids total de l'émulsion.

L'invention se rapporte aussi à une crème pour le traitement de la peau contenant une émulsion telle que définie précédemment.

L'invention a encore pour objet l'utilisation de l'émulsion définie précédemment pour la préparation d'une crème destinée au traitement de maladies de peau.

Des exemples concrets d'émulsion mais nullement limitatifs illustrant l'invention vont maintenant être donnés.

Dans ces exemples, les émulsions ont été obtenues en incorporant les premier et second esters dans de l'eau contenant éventuellement les additifs hydrophiles, en chauffant ensuite l'ensemble entre 60° C et 70° C sous agitation pendant 15 à 45 min. Après dissolution complète des esters, on introduit dans la phase aqueuse la phase huileuse préalablement chauffée à 60° C et contenant les additifs lipophiles. On agite alors violemment l'ensemble en maintenant le chauffage. On laisse refroidir l'ensemble sous agitation modérée.

Les exemples 1 et 2 donnent des émulsions blanches, brillantes, douces, à application légère qui pénètrent facilement. Après application, la peau brille et colle peu. Les émulsions de ces deux exemples sont destinées au soin des peaux sèches.

### EXEMPLE 1 :

| *Emulsionnants* | |
|---|---|
| - Sesquistéarate de méthylglucose (GRILLOCOSE PS) | 3 % |
| - Palmito-stéarate de saccharose (CRODESTA F160) (73 % mono-ester, 27 % di-triester) | 6 % |

| *Phase huileuse* | |
|---|---|
| - Huile de vaseline | 20 % |
| - Myristate d'isopropyle | 25 % |

| *Additifs* | |
|---|---|
| - Antioxydants (BHT et BHA) | 0,5 % |
| - Conservateurs (Parabenzoate de méthyle et Imidazolidinyl urée) | 0,1 % |
| - Co-émulsionnant (alcool cétylique) | 1 % |
| *Eau* | qsp 100 % |

### EXEMPLE 2 : Crème nourrissante

| *Emulsionnants* | |
|---|---|
| - Sesquistéarate de méthylglucose (GRILLOCOSE PS) | 3,5 % |
| - Stéarate de saccharose (CRODESTA F110) (52 % mono-ester, 48 % di-, triester) | 1,5 % |

| *Phase huileuse* | |
|---|---|
| - Huile de tournesol | 15 % |
| - Huile de jojoba | 4 % |

| *Additifs* | |
|---|---|
| - Alcool de lanoline | 3 % |
| - Gomme de xanthane | 0,2 % |
| - Antioxydants (BHT et BHA) | 0,7 % |
| - Conservateurs (Paraben et Imidazolinyl urée) | 0,2 % |
| - Co-émulsionnant (alcool béhénique) | 2 % |
| *Eau* | qsp 100 % |

### EXEMPLE 3 :

| *Emulsionnants* | |
|---|---|
| - Palmitate de glucose (décrit dans la demande de brevet français n° 92-03811) | 3 % |
| - Palmito-stéarate de saccharose (CRODESTA F70) (39 % mono-ester ; 61 % di-triester) | 7 % |

| *Phase huileuse* | |
|---|---|
| - Perhydrosqualène | 5 % |
| - Isostéarate d'isostéaryle | 13 % |

| *Additifs* | |
|---|---|
| - Glycérol | 3 % |
| - Antioxydants (BHT et BHA) | 0,6 % |
| - Conservateurs (Paraben et Imidazolinyl urée) | 0,15 % |
| *Eau* | qsp 100 % |

L'émulsion obtenue est blanche et brillante et sert à la protection de la peau comme l'exemple 1, tout en étant moins grasse.

### EXEMPLE 4 :

| *Emulsionnant* | |
|---|---|
| - Palmitate d'éthylglucose (BIOSURF16) | 6 % |
| - Mono-laurate de saccharose (CRODESTA SL 40) | 4 % |

| *Phase huileuse* | |
|---|---|
| - Polydiméthyl siloxane (ABIL 10 vendu par la Société GOLDSCHMIDT) | 2 % |
| - Polycétylméthyl siloxane (ABIL WAX 9801 vendu par la Société GOLDSCHMIDT) | 2 % |
| - Emollient (Pur Sellin Liquide vendu par la Société DRAGOCO) | 2 % |

| *Additifs* | |
|---|---|
| - Antioxidants (BHT et BHA) | 0,55 % |
| - Conservateurs (Parabenzoate de méthyle et Imidazolinyl urée) | 0,05 % |
| *Eau* | qsp 100 % |

L'exemple 4 donne une émulsion blanche et brillante, destinée au soin des peaux normales.

Aucune des émulsions des exemples 1 à 4 ne comprend de parfum. En effet, un des avantages de l'invention est que ces émulsions ne présentent aucune odeur désagréable contrairement à des émulsions qui comprennent comme agent émulsionnant des dérivés de pétrole. Il n'est donc pas nécessaire dans les émulsions selon l'invention d'ajouter un parfum pour contrecarrer la présence de ces odeurs désagréables.

Les propriétés sensorielles de la crème de l'exemple 2 ont été testées sur un panel de 30 volontaires experts. Chacun a évalué la crème en effectuant une mono-application sur le visage et en remplissant un questionnaire faisant figurer 19 descripteurs ayant trait à l'aspect du produit, à ses qualités de prise, d'étalement, de confort immédiat et dans le temps, après une journée.

Une crème "contre-exemple" conforme à l'art antérieur dans laquelle le mélange des esters de glucose et de saccharose (5 % en poids au total) est remplacé par uniquement de l'ester de glucose (5 % en poids) a été testée dans les mêmes conditions.

La rugosité des crèmes lors de l'application sur la peau peut être évaluée par les descripteurs souplesse, cireux et légèreté. La crème de l'exemple 2 est jugée plus souple (significativité selon le test de STUDENT ; risque α = 0,01 %), plus légère (significativité selon le test de STUDENT ; risque α = 0,01 %), moins cireuse (significativité selon le test de STUDENT ; risque α = 0,01 %), que la crème "contre-exemple".

L'opinion générale est également en faveur de la crème de l'exemple 2 (intervalle de confiance α = 10 %) ; cette dernière obtient un score de 16 % d'opinions satisfaisantes de plus que celui de la crème contenant uniquement l'ester de glucose.

## Revendications

1. Emulsion huile-dans-eau comprenant une phase huileuse dispersée dans une phase aqueuse, un système émulsionnant, caractérisée en ce que le système émulsionnant contient au moins un premier ester choisi parmi les esters d'acide gras et de glucose et les esters d'acide gras et d'alkylglucose et au moins un second ester d'acide gras et de saccharose.

2. Emulsion selon la revendication précédente, caractérisée en ce que la phase aqueuse représente de 40 % à 94 % en poids par rapport au poids total de l'émulsion.

3. Emulsion selon l'une des revendications précédentes, caractérisée en ce que la phase huileuse représente de 5 % à 50 % en poids par rapport au poids total de l'émulsion.

4. Emulsion selon l'une des revendications précédentes, caractérisée en ce que le système émulsionnant représente de 1 % à 10 % en poids par rapport au poids total de l'émulsion.

5. Emulsion selon l'une des revendications précédentes, caractérisée en ce que la phase huileuse représente de 10 % à 30 % en poids par rapport au poids total de l'émulsion.

6. Emulsion selon l'une des revendications précédentes, caractérisée en ce qu'elle contient en poids :
- de 66 % à 86 % de phase aqueuse,
- de 10 % à 30 % de phase huileuse,
- de 4 % à 6 % de système émulsionnant,
- de 0 % à 10 % d'additifs.

7. Emulsion selon l'une des revendications précédentes, caractérisée en ce que le rapport premier ester d'acide gras/second ester d'acide gras est choisi dans les rapports 70/30 et 30/70.

8. Emulsion selon l'une des revendications précédentes, caractérisée en ce que ce rapport est de 50/50.

9. Emulsion selon l'une des revendications précédentes, caractérisée en ce que le premier ester d'acide gras est choisi parmi les composés obtenus par réaction d'un acide gras à chaîne carbonée saturée ou insaturée ayant de 12 à 22 atomes de carbone avec du glucose ou avec un alkylglucose dont le groupement alkyle contient de 1 à 6 atomes de carbone.

10. Emulsion selon la revendication 9, caractérisée en ce que le premier ester d'acide gras contient un mélange de dérivés mono-, di-, tri-, tétra-ester avec une proportion d'au moins 50 % en poids de dérivés mono- et di-ester n'excédant pas 95 % en poids de monoester par rapport au poids total du mélange.

11. Emulsion selon l'une des revendications précédentes, caractérisée en ce que le second ester d'acide gras est choisi parmi les composés obtenus par réaction d'acide gras à chaîne carbonée saturée ou insaturée ayant de 12 à 22 atomes de carbone avec du saccharose.

12. Emulsion selon la revendication 11, caractérisée en ce que le second ester d'acide gras contient un mélange du dérivé mono-, di-, tri-, tétra-et polyester avec une proportion en dérivés monoester allant de 30 % à 95 % en poids par rapport au poids total du mélange.

13. Emulsion selon l'une des revendications précédentes, caractérisée en ce qu'elle contient un additif choisi parmi les alcools gras, les agents gélifiants, les hydratants, les vitamines, les parfums, les conservateurs, les anti-oxydants.

14. Emulsion selon l'une quelconque des revendications précédentes, caractérisée en ce que le premier ester est choisi parmi le sesquistéarate de méthylglucose, le palmitate d'éthylglucose, de méthylglucose, le palmitate de glucose.

15. Emulsion selon l'une des revendications précédentes, caractérisée en ce que le second ester est choisi parmi le palmito-stéarate de saccharose, le stéarate de saccharose et le mono laurate de saccharose.

16. Crème pour le traitement de la peau, caractérisée en ce qu'elle contient une émulsion selon l'une quelconque des revendications précédentes.

17. Utilisation de l'émulsion selon l'une quelconque des revendications 1 à 15 pour la préparation d'une crème destinée au traitement des maladies de peau.

18. Utilisation d'au moins un premier ester choisi parmi les esters d'acide gras et de glucose et les esters d'acide gras et d'alkylglucose et d'au moins un second ester d'acide gras et de saccharose, dans une émulsion huile-dans-eau, comme système émulsionnant.

19. Utilisation selon la revendication 18, caractérisée en ce que le premier ester d'acide gras est choisi parmi les composés obtenus par réaction d'un acide gras à chaîne carbonée saturée ou insaturée ayant de 12 à 22 atomes de carbone avec du glucose ou avec un alkylglucose dont le groupement alkyle contient de 1 à 6 atomes de carbone.

20. Utilisation selon la revendication 18 ou 19, caractérisée en ce que le second ester d'acide gras est choisi parmi les composés obtenus par réaction d'acide gras à chaîne carbonée saturée ou insaturée ayant de 12 à 22 atomes de carbone avec du saccharose.

## Claims

1. Oil-in-water emulsion comprising an oily phase dispersed in an agueous phase and an emulsifying system, characterized in that the emulsifying system contains at least one first ester chosen from the fatty acid esters of glucose and the fatty acid esters of alkylglucose and at least one second fatty acid ester of sucrose.

2. Emulsion according to the preceding claim, characterized in that the agueous phase represents from 40 % to 94 % by weight relative to the total weight of the emulsion.

3. Emulsion according to Claim 1, characterized in that the oily phase represents from 5 % to 50 % by weight relative to the total weight of the emulsion.

4. Emulsion according to Claim 1, characterized in that the emulsifying system represents from 1 % to 10 % by weight relative to the total weight of the emulsion.

5. Emulsion according to Claim 1, characterized in that the oily phase represents from 10 % to 30 % by weight relative to the total weight of the emulsion.

6. Emulsion according to Claim 1, characterized in that it contains by weight:
- from 66 % to 86 % of aqueous phase,
- from 10 % to 30 % of oily phase,
- from 4 % to 6 % of emulsifying system,
- from 0 % to 10 % of additives.

7. Emulsion according to Claim 1, characterized in that the first fatty acid ester/second fatty acid ester ratio is chosen between the ratios 70/30 and 30/70.

8. Emulsion according to Claim 7, characterized in that this ratio is 50/50.

9. Emulsion according to Claim 1, characterized in that the first fatty acid ester is chosen from the compounds obtained by reacting a fatty acid of saturated or unsaturated carbon chain having from 12 to 22 carbon atoms with glucose or with an alkylglucose in which the alkyl group contains from 1 to 6 carbon atoms.

10. Emulsion according to Claim 9, characterized in that the first fatty acid ester contains a mixture of mono-, di-, tri- and tetraester derivatives with a proportion of at least 50 % by weight of mono- and diester derivatives not exceeding 95 % by weight of monoester relative to the total weight of the mixture.

11. Emulsion according to Claim 1, characterized in that the second fatty acid ester is chosen from the compounds obtained by reacting a fatty acid of saturated or unsaturated carbon chain having from 12 to 22 carbon atoms with sucrose.

12. Emulsion according to Claim 11, characterized in that the second fatty acid ester contains a mixture of the mono-, di-, tri-, tetra- and polyester derivatives with a proportion of monoester derivatives ranging from 30 % to 95 % by weight relative to the total weight of the mixture.

13. Emulsion according to Claim 1, characterized in that it contains an additive chosen from fatty alcohols, gelling agents, hydrating agents, vitamins, fragrances, preserving agents and antioxidants.

14. Emulsion according to Claim 1, characterized in that the first ester is chosen from methylglucose sesquistearate, ethylglucose palmitate, methylglucose palmitate and glucose palmitate.

15. Emulsion according to Claim 1, characterized in that the second ester is chosen from sucrose palmitostearate, sucrose stearate and sucrose monolaurate.

16. Cream for treating the skin, characterized in that it contains an emulsion according to Claim 1.

17. Use of the emulsion according to Claim 1 for the preparation of a cream intended for treating skin diseases.

18. Use of at least one first ester chosen from fatty acid esters of glucose and fatty acid esters of alkylglucose and at least one second faty acid ester of sucrose, in an oil-in-water emulsion, as an emulsifying system.

19. Use according to Claim 18, characterized in that the first fatty acid ester is chosen from the compounds obtained by reacting a fatty acid of saturated or unsaturated carbon chain having from 12 to 22 carbon atoms with glucose or with an alkylglucose in which the alkyl group contains from 1 to 6 carbon atoms.

20. Use according to Claim 18, characterized in that the second fatty ester is chosen from the compounds obtained by reacting a fatty acid of saturated or unsaturated carbon chain having from 12 to 22 carbon atoms with sucrose.

## Patentansprüche

1. Öl-in-Wasser-Emulsion, die eine in einer wäßrigen Phase emulgierte Ölphase und ein Emulgatorsystem umfaßt,
dadurch **gekennzeichnet**, daß
das Emulgatorsystem mindestens einen ersten Ester, der unter Glucose-Fettsäureestern und Alkylglucose-Fettsäureestern ausgewählt ist, und mindestens einen zweiten Ester aus einer Fettsäure und Saccharose enthält.

2. O/W-Emulsion nach dem vorhergehenden Anspruch,
dadurch gekennzeichnet, daß
der Anteil der wäßrigen Phase 40 bis 94 Gew.-%, bezogen auf das Gesamtgewicht der Emulsion, beträgt.

3. O/W-Emulsion nach einem der vorhergehenden Ansprüche,
dadurch gekennzeichnet, daß
der Anteil der Ölphase 5 bis 50 Gew.-%, bezogen auf das Gesamtgewicht der Emulsion, beträgt.

4. O/W-Emulsion nach einem der vorhergehenden Ansprüche,
dadurch gekennzeichnet, daß
der Anteil des Emulgatorsystems 1 bis 10 Gew.-%, bezogen auf das Gesamtgewicht der Emulsion, beträgt.

5. O/W-Emulsion nach einem der vorhergehenden Ansprüche,
dadurch gekennzeichnet, daß
der Anteil der Ölphase 10 bis 30 Gew.-%, bezogen auf das Gesamtgewicht der Emulsion, beträgt.

6. O/W-Emulsion nach einem der vorhergehenden Ansprüche,
dadurch gekennzeichnet, daß
sie enthält:
- 66 bis 86 Gew.-% wäßrige Phase,
- 10 bis 30 Gew.-% Ölphase,
- 4 bis 6 Gew.-% Emulgatorsystem,
- 0 bis 10 % Zusätze.

7. O/W-Emulsion nach einem der vorhergehenden Ansprüche,
dadurch gekennzeichnet, daß
das Verhältnis erster Fettsäureester/zweiter Fettsäureester im Bereich von 70/30 bis 30/70 ausgewählt ist.

8. O/W-Emulsion nach einem der vorhergehenden Ansprüche,
dadurch gekennzeichnet, daß
dieses Verhältnis 50/50 beträgt.

9. O/W-Emulsion nach einem der vorhergehenden Ansprüche,
dadurch gekennzeichnet, daß
der erste Fettsäureester unter Verbindungen ausgewählt ist, die durch Umsetzung einer Fettsäure mit gesättigter oder ungesättigter Kohlenstoffkette, die 11 bis 22 Kohlenstoffatome aufweist, mit Glucose oder einer Alkylglucose, deren Alkylgruppe 1 bis 6 Kohlenstoffatome enthält, erhältlich sind.

10. O/W-Emulsion nach Anspruch 9,
dadurch gekennzeichnet, daß
der erste Fettsäureester ein Gemisch von Mono-, Di-, Tri- und Tetraesterderivaten enthält, wobei der Gehalt an Mono- und Diesterderivaten mindestens 50 Gew.-% und der Gehalt an Monoester nicht mehr als 95 Gew.-%, bezogen auf das Gesamtgewicht des Gemischs, beträgt.

11. O/W-Emulsion nach einem der vorhergehenden Ansprüche,
dadurch gekennzeichnet, daß
der zweite Fettsäureester unter den Verbindungen ausgewählt ist, die durch Umsetzung einer Fettsäure mit gesättigter oder ungesättigter Kohlenstoffkette, die 12 bis 22 Kohlenstoffatome aufweist, mit Saccharose erhältlich sind.

12. O/W-Emulsion nach Anspruch 11,
dadurch gekennzeichnet, daß
der zweite Fettsäureester ein Gemisch von Mono-, Di-, Tri-, Tetra- und Polyestern enthält, wobei der Gehalt an Monoesterderivaten 30 bis 95 Gew.-%, bezogen auf das Gesamtgewicht, beträgt.

13. O/W-Emulsion nach einem der vorhergehenden Ansprüche,
dadurch gekennzeichnet, daß
sie einen Zusatz enthält, der unter Fettalkoholen, Gelbildnern, Hydratisierungsmitteln, Vitaminen, Parfüms, Konservierungsmitteln und Antioxidantien ausgewählt ist.

14. O/W-Emulsion nach einem der vorhergehenden Ansprüche,
dadurch gekennzeichnet, daß
der erste Ester unter Methylglucosesesquistearat, Ethylglucosepalmitat, Methylglucosepalmitat und Glucosepalmitat ausgewählt ist.

15. O/W-Emulsion nach einem der vorhergehenden Ansprüche,
dadurch gekennzeichnet, daß
der zweite Ester unter Saccharosepalmitatstearat, Saccharosestearat und Saccharosemonolaurat ausgewählt ist.

16. Creme zur Behandlung der Haut
dadurch gekennzeichnet, daß
sie eine Emulsion nach einem der vorhergehenden Ansprüche enthält.

17. Verwendung der Emulsion nach einem der Ansprüche 1 bis 15 zur Herstellung einer Creme, die für die Behandlung von Hautkrankheiten bestimmt ist.

18. Verwendung mindestens eines ersten Esters, der unter Glucose-Fettsäureestern und Alkylglucose-Fettsäureestern ausgewählt ist, und mindestens eines zweiten Esters aus einer Fettsäure und Saccharose als Emulgatorsystem in Öl-in-Wasser-Emulsionen.

19. Verwendung nach Anspruch 18,
dadurch gekennzeichnet, daß
der erste Fettsäureester unter den Verbindungen ausgewählt wird, die durch Umsetzung einer Fettsäure mit einer gesättigten oder ungesättigten Kohlenstoffkette, die 12 bis 22 Kohlenstoffatome aufweist, mit Glucose oder einer Alkylglucose, deren Alkylgruppe 1 bis 6 Kohlenstoffatome enthält, erhältlich sind.

20. Verwendung nach Anspruch 18 oder 19,
dadurch gekennzeichnet, daß
der zweite Fettsäureester unter den Verbindungen ausgewählt wird, die durch Umsetzung einer Fettsäure mit gesättigter oder ungesättigter Kohlenstoffkette, die 12 bis 22 Kohlenstoffatome aufweist, mit Saccharose erhältlich sind.
